# DEMANDE DE BREVET EUROPEEN

(11) **EP 1 275 401 A1**
(43) Date de publication de la demande: **15.01.2003**
(21) Numéro de dépôt: 02291689.4
(22) Date de dépôt: 05.07.2002
(51) Int. Cl.: A61L 2/23, A01N 37/16

(54) **Composition à base de composé peroxyde et de composé N-acyle et procédé de désinfection**

(30) Priorité: 10.07.2001 FR 0109129
(71) Demandeur: Laboratoires Rivadis, 79100 Thouars (FR)
(72) Inventeur: Larnaudie, Nathalie Chrystine, 79100 Thouars (FR); Cottron, Olivier, Pierre, 49300 Cholet (FR)
(74) Mandataire: Eidelsberg, Olivier Nathan

(57) **Abrégé**

Cette composition comprend à l'état solide un composé peroxydé, un composé N-acylé et un acide passant le test A, par exemple l'acide sorbique. Mélangée à de l'eau, elle a une action antimicrobienne.

## Description

La présente invention se rapporte aux compositions et procédés de désinfection notamment d'objets ou de surfaces vivants ou inertes, par exemple dans le domaine médical.

A la demande de brevet international WO98/25468 on décrit un procédé de désinfection d'instruments médicaux. On prépare la composition en deux stades. Dans un premier stade on fait réagir de l'eau oxygénée ou des composés peroxydés qui forment de l'eau oxygénée dans l'eau en milieu alcalin aqueux sur des composés N-acylés qui dans ces conditions forment un péracide. Dans un second stade on abaisse le pH de la composition obtenue au stade précédent à l'aide d'acides habituels, ce qui augmente extraordinairement l'efficacité anti-microbienne, tout en ayant une faible corrosion.

Cette manière de procéder a deux inconvénients. Elle est compliquée et fastidieuse par la mise en oeuvre de deux stades opératoires successifs. Elle est dangereuse parce qu'elle nécessite un transvasement d'acide, la précision de la quantité à transvaser étant également source de difficulté.

L'invention remédie à ces inconvénients par une composition qui ne nécessite pour devenir active que d'y verser de l'eau en un seul stade.

La composition suivant l'invention est à l'état solide et comprend un composé peroxydé, un composé N-acylé apte à réagir en milieu aqueux alcalin sur le composé peroxydé pour donner, par une réaction de perhydrolyse, un peracyle, et un acide tel qu'une solution S telle que définie dans le test A de l'acide en question présente, pour une valeur du pH à l'équilibre final donnée comprise entre 7 et 7,9, une différence des pH mesurés à l'instant t = 30 secondes et à l'instant t = 30 minutes qui est supérieure à 0,6.

Dans cette composition l'acide est certes soluble dans l'eau, mais sa vitesse de dissolution est lente et notamment bien plus lente que la vitesse à laquelle se déroule en milieu aqueux et alcalin la réaction entre le composé peroxydé et le composé N-acylé. Cette réaction s'effectue ainsi alors que l'acide n'est pas encore dissout ou ne l'est que très peu et donc à un pH basique notamment de 11 à 8,0 où la réaction s'effectue bien. Après que la réaction a eu le temps de s'effectuer, la dissolution plus lente de l'acide suivant l'invention permet de diminuer peu à peu le pH pour l'amener à une valeur inférieure à 8,0 (ce qui correspond au pk_{A} a de l'acide péracétique) où l'activité biocide de la composition est la meilleure.

Suivant un mode de réalisation préféré de l'invention, l'acide est un acide mono ou di-alcénoïque ayant un minimum de 4 atomes de carbones et solide à température ambiante, un dérivé de ces acides, l'acide benzoïque, un dérivé de ce dernier ou un mélange de ceux-ci.

Le composé peroxydé peut être un adduit d'eau oxygénée et de divers supports que l'on désigne également éventuellement sous le nom de perhydrate. Ce peut être un perhydrate d'urée, un perhydrate de citrate de sodium ou un perhydrate de carbonate de sodium (Na₂CO₃ x 1,5 H₂O) x 1,5 H2O) désigné habituellement aussi comme étant du percarbonate de sodium. On peut aussi utiliser des composés peroxo-minéraux qui s'hydrolysent spontanément dans l'eau comme le perborate de sodium, notamment le monohydrate de perborate de sodium et le tétrahydrate de perborate de sodium. On préfère tout particulièrement utiliser dans le procédé suivant l'invention le perborate de sodium monohydraté. On peut bien entendu utiliser plusieurs composés peroxydés en mélange.

Le composé N-acylé que l'on utilise dans la composition suivant l'invention est notamment un composé acylé qui comporte sur l'atome d'azote, qui porte le groupe acyle, un autre groupe céto et/ou dans lequel l'atome d'azote fait partie d'un système hétérocyclique ou supporte deux radicaux alkyles. Comme composés N-acylé qui conviennent on peut citer les alcoylènediamine plusieurs fois acylées comme par exemple la tétraacétyléthylènediamine, les glycoluriles acylés et notamment le tétraacétyglycolurile, les hydantoine N-acylées, les hydrazides, les triazoles, les triazines, les urazoles, les dicétopipérazines les sulfurylamides, les lactames et les cyanurates. On préfère tout particulièrement la tétraacétyléthylénediamine (T.A.E.D.) et également le trétaacétygliculurile (T.A.G.U.) ainsi que la 1,5-diacétyl-z, 4-dioxohexahydro 1,3, 5 triazine (D.A.D.H.). On peut aussi utiliser plusieurs composés N-acylés.

L'acide monoalcénoique ou dialcénoique que l'on peut utiliser est un acide qui est solide à la température ambiante ou au moins à la température à laquelle on prépare la solution désinfectante en versant de l'eau dans la composition suivant l'invention et dont la vitesse de dissolution est lente. On préfère tout particulièrement les acides dialcénoiques et notamment l'acide sorbique ou acide trans, trans-2,4-, hexadiènoique.

On peut ajouter à la composition suivant l'invention des additifs utiles pour l'utilisation de cette composition en vue de désinfecter des instruments médicaux. On peut notamment lui ajouter des agents tensioactifs, des agents complexants pour lutter contre la dureté de l'eau, des agents complexants d'ions métalliques lourds et des sels minéraux solubles dans l'eau.

On peut utiliser notamment des inhibiteurs de corrosion et des agents tensioactifs. La quantité de ces additifs peut varier dans de très grandes limites mais elle ne dépasse pas habituellement 20 % en poids environ et de préférence 10 % du poids total de la composition.

Comme agent tensioactif on peut utiliser des agents tensioactifs anionique et non ionique mais également des agents tensioactifs cationiques et amphothères. On préfère des agents tensioactifs anioniques et non ioniques ainsi que des mélanges de plusieurs agents tensioactifs de ces deux classes. On peut citer notamment des alcoylbenzènesulfonates, des alcoylsulfate qui sont les sels de d'hémi-esters de l'acide sulfurique d'alcools à longue chaîne, des alkyléthersulfates qui sont des sels d'hémiesters de l'acide sulfurique de l'acide sulfurique d'alcools alcoxylés, et notamment éthoxyles, à longue chaîne, des alcanesulfonates et des oléfinesulfonates. Des agents tensioactifs anioniques sont de préférence utilisés sous la forme de sels de sodium. Des agents tensioactifs non ioniques qui conviennent particulièrement sont les alcools à longues chaînes alcoxylés les alkylbenzènesulfonates de sodium et les alkylsulfate de sodium.

Comme agent complexant d'ion de métaux lourds, on envisage en premier lieu des acides aminopolycarboxyliques ou leurs sels, par exemple l'acide éthylènediaminetétracétique, mais également des acides aminopolyphosphoniques comme l'acide éthyldiaminotétraméthylènephosphonique ou également l'acide hydroxyéthanédiphosphonique, ou également l'acide 1-hydroxyethylidène -1,1- disphosphonique et leurs sels.

On peut ajouter également un colorant et un parfum. La composition comprend pour une mole de composé péroxydé de 1,5 à 2,5 moles de composé N-acylé.

On utilise la composition suivant l'invention de la manière suivante. On verse de 90 à 99 parties en poids d'eau pour une partie en poids de la composition et on laisse réagir pendant au moins 10 minutes et de préférence pendant 15 minutes. Pour désinfecter des instruments, on immerge les instruments dans la solution entre 5 minutes et 1 heures en fonction de l'agent infectieux.

### Définition du test A

Le test A a pour objet de sélectionner les acides organiques qui permettent l'acidification du milieu réactionnel en un seul stade suivant l'invention.

Ces acides doivent notamment conduire à des valeurs de pH inférieures à 8, où l'activité biocide est la meilleure.

Sous faible agitation, on dissout la composition désinfectante dans l'eau tout en mesurant l'évolution du pH au cours du temps.

La formule de base du mélange S de test d'un acide A donné est : TAED (Warwick) 0,3 % Perborate de sodium monohydraté (Solvay) 0,4 %, Lauryl sulfate de sodium (Cognis) 0,01 %, Benzotriazole (Société chimique de Montville) 0,1 %, Sel tetrasodique de l'acide 1-hydroxyethylidène -1, 1 diphosponique (Solutia) 0,1 %, Acide x % et Eau déminéralisée qsp 100 % (les pourcentages étant en poids). x est choisi de sorte que le pH de la solution finale à un instant théoriquement infini (une fois que tout l'acide a été dissout) soit égal à une valeur donnée, ici 7,9 et 7,0, pour un volume de 500 ml de solution dans l'eau.

La détermination de x peut se faire soit par les calculs sur la base des poids moléculaires respectifs et des équations d'équilibre et de masse, soit de manière expérimentale par tâtonnements, notamment dichotomiques.

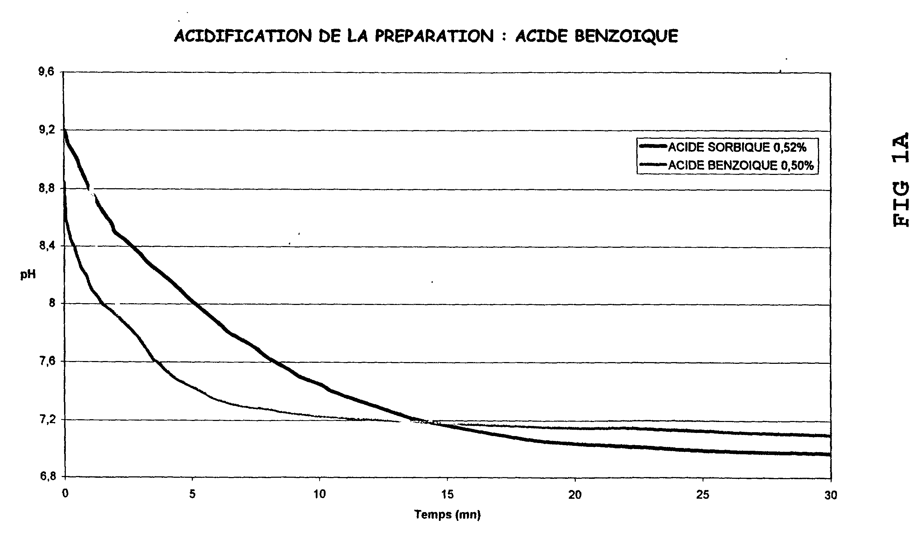

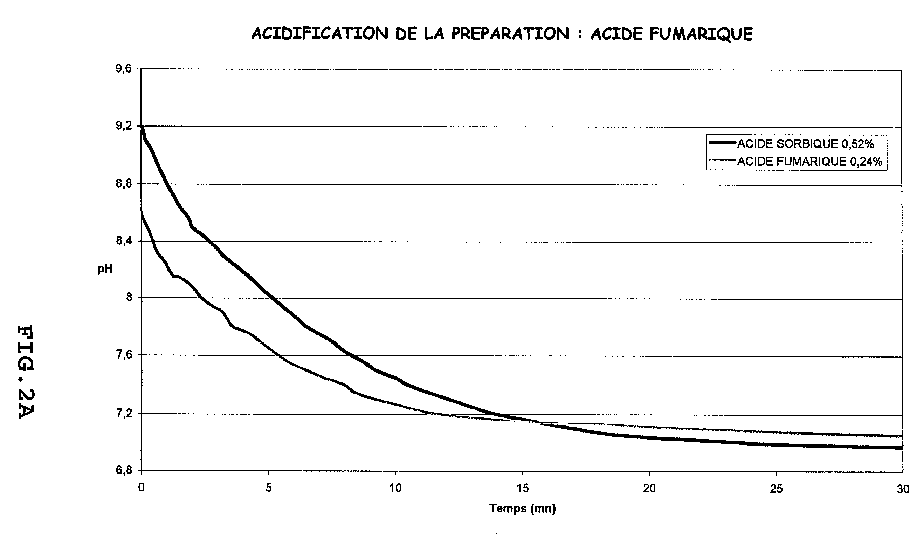

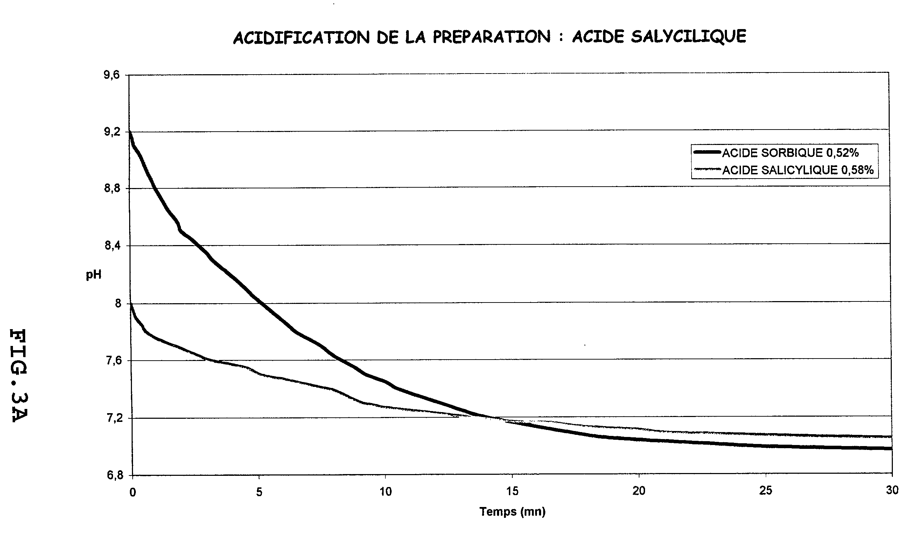

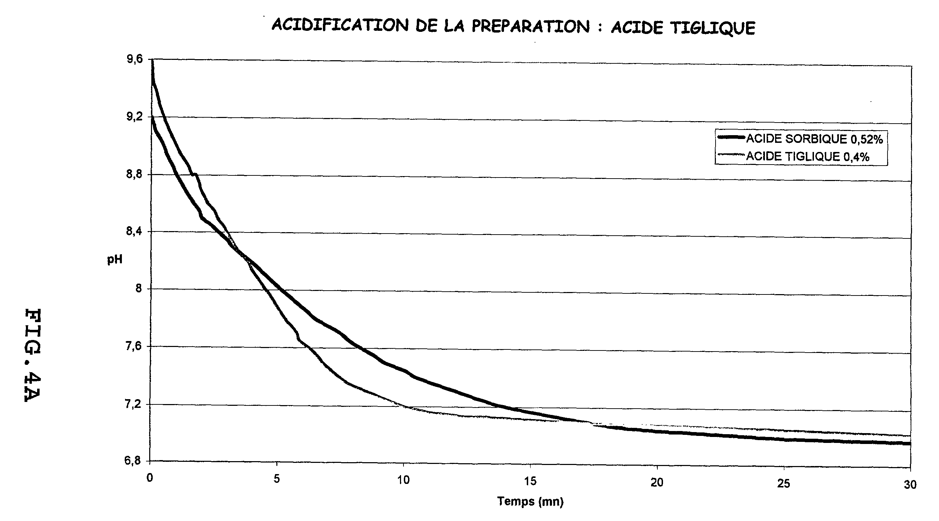

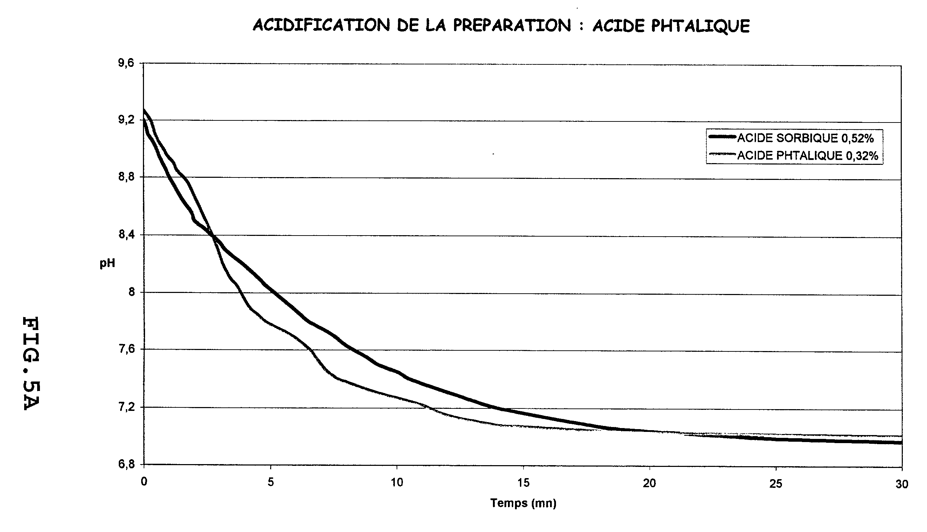

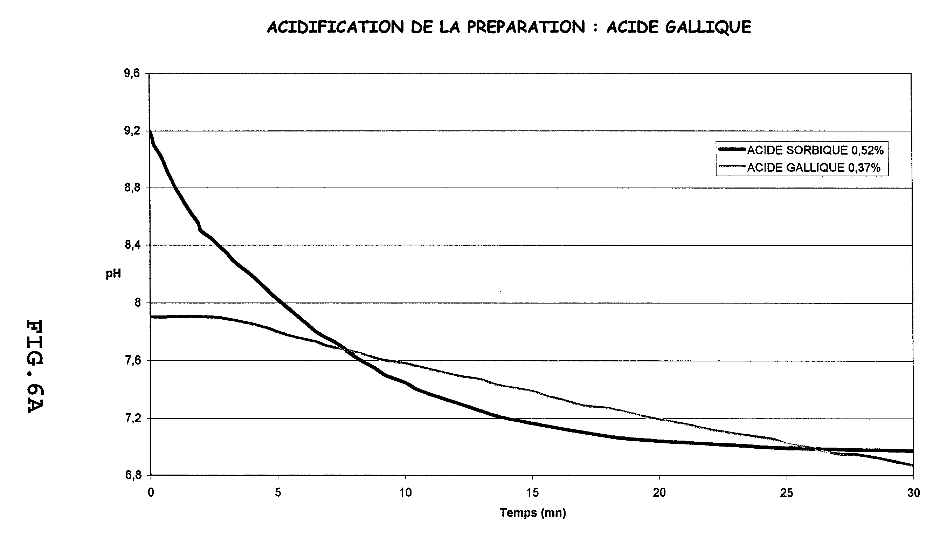

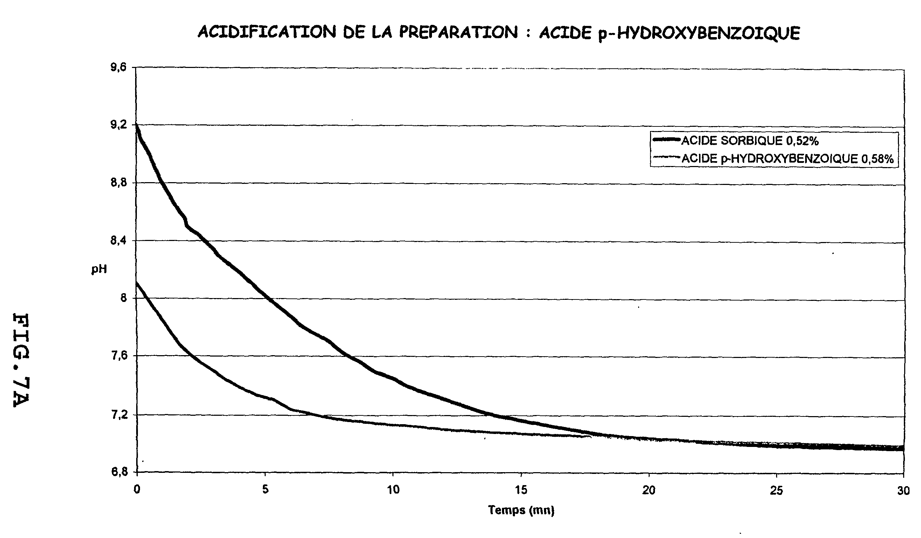

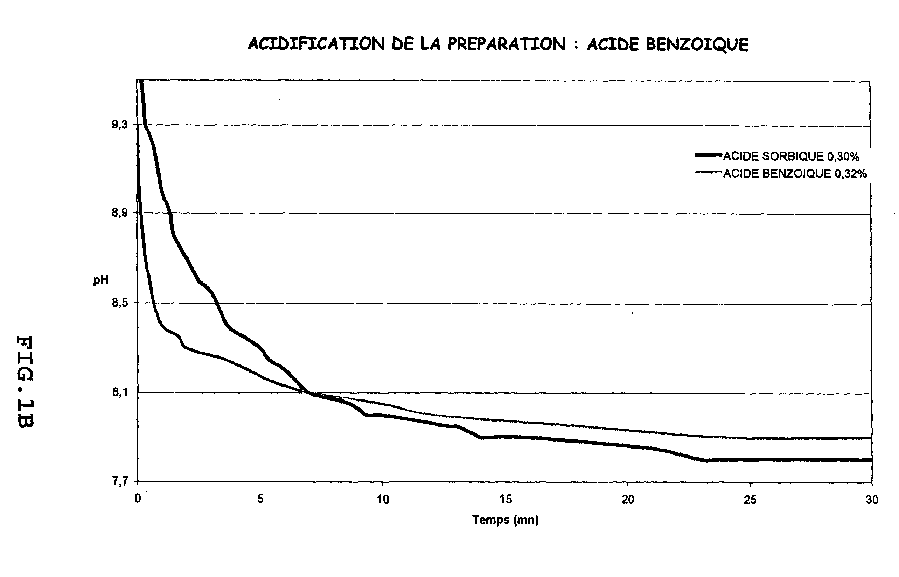

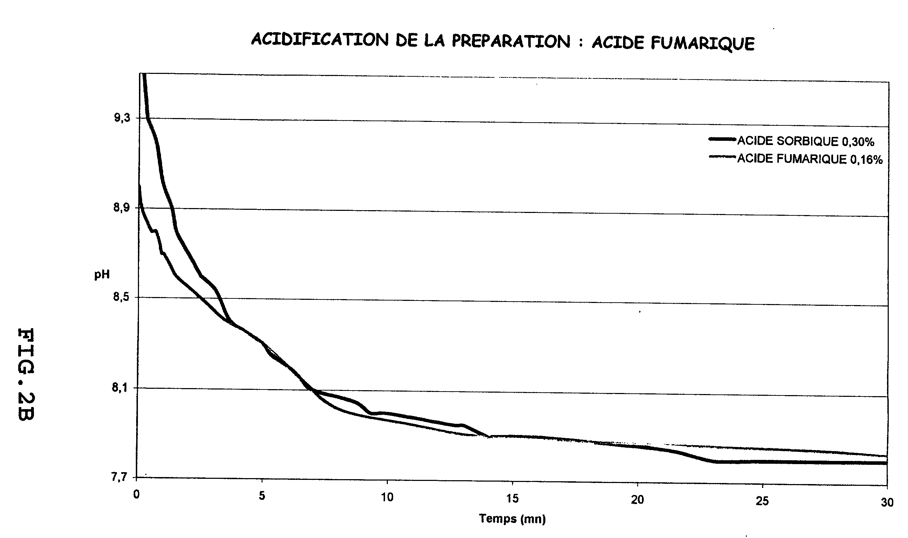

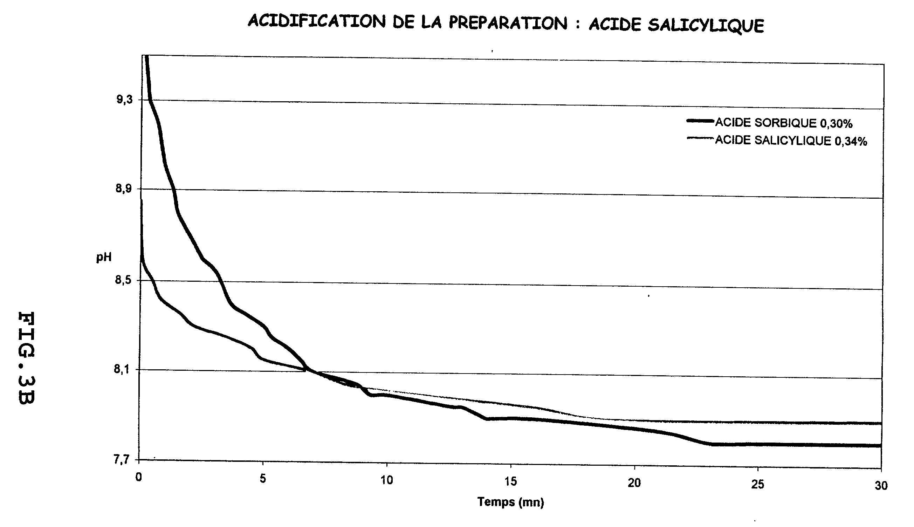

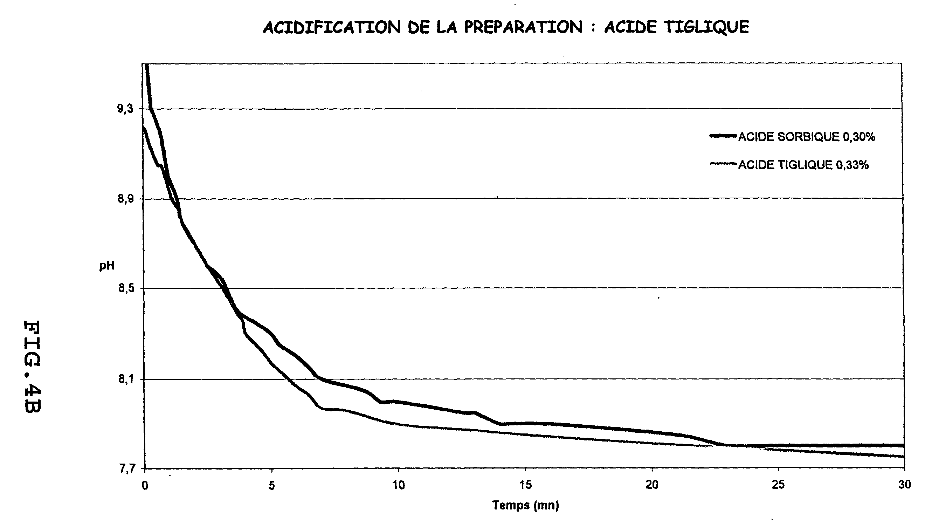

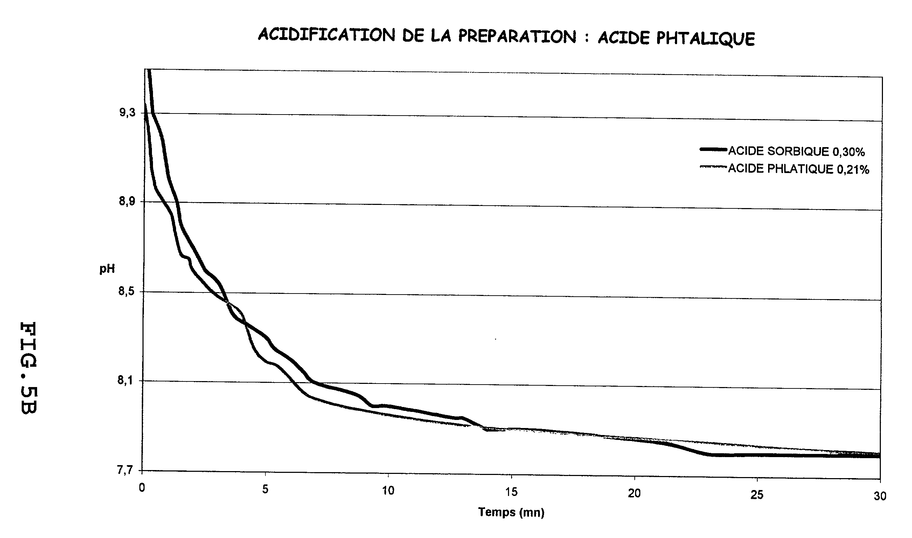

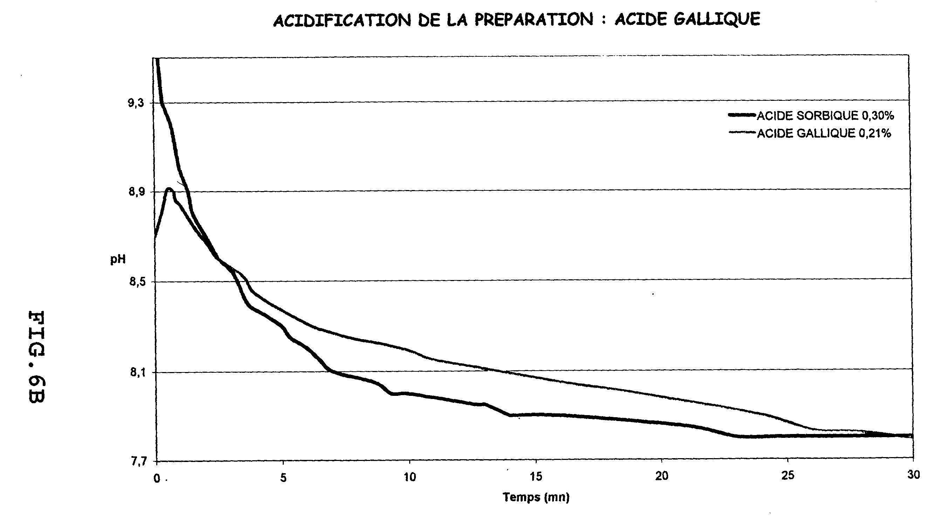

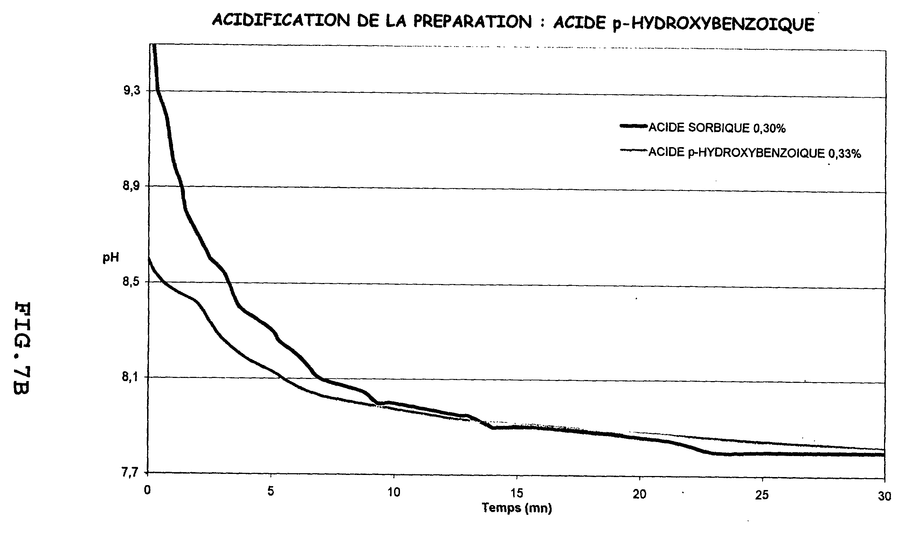

On introduit le mélange S de test pour un acide donné dans un becher muni d'un barreau aimanté à section triangulaire enrobé PTFE (⌀ cercle circonscrit = 12 mm). On place le becher sur l'agitateur magnétique réglé à la vitesse de 100t/mn et on positionne l'électrode à PH (PH-neutre 763 Multi-Calimatic (Knick) avec son électrode combiné pH/température corps verre (Ag/Agcl) (Broblock Scientific). On introduit rapidement la quantité définie d'eau déminéralisée (pour un volume final de solution de 500 ml) et on déclenche le chronomètre. On relève les valeurs du PH au cours du temps. On obtient alors une courbe caractéristique, pour un pH final donné, de l'acide en question. Les figures la à 7a représentent ces courbes caractéristiques respectivement pour l'acide benzoïque, l'acide fumarique, l'acide salycilique, l'acide tiglique, l'acide phtalique, l'acide gallique et l'acide p-hydroxybenzoïque pour un pH final de 7 et les figures 1b à 7b représentent ces mêmes courbes pour les mêms acides pour un ph final de 7,9. Dans toutes les figures, il est représenté en gras la courbe correspondant à l'acide sorbique et en trait clair la courbe de l'acide donné.

Un acide passe le test A (c'est-à-dire convient suivant l'invention) si la différence des pH mesurés à 30 secondes et à 30 minutes reste supérieure à 0,6.

Les résultats du test A pour les acides dont les courbes caractéristiques sont représentées aux figures peuvent être résumés comme suit

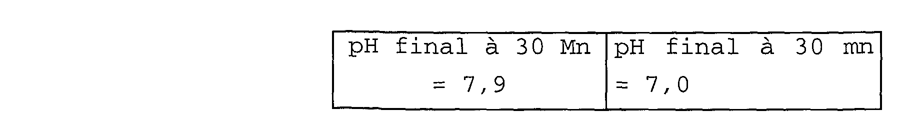

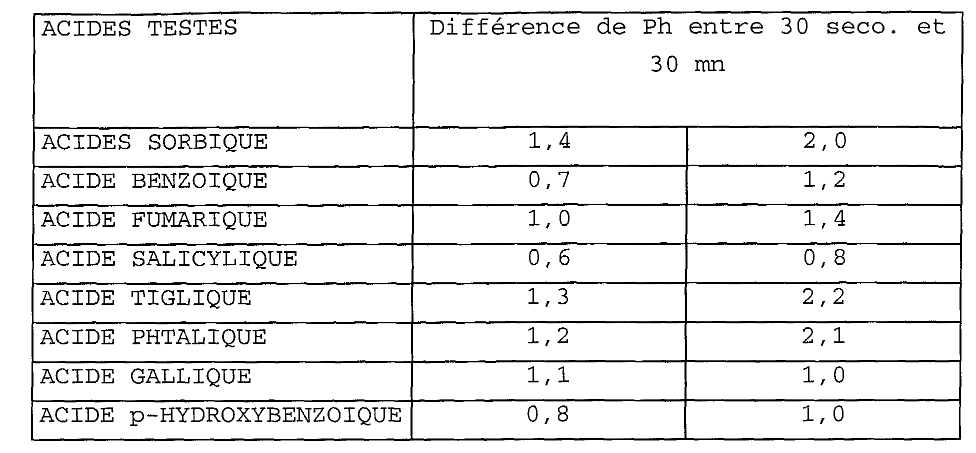

Les pourcentages en poids des acides sont indiqués aux figures; à savoir: acide sorbique (0,52 % ; 0,30 %), acide benzoïque (0,5 ; 0,32), acide fumarique (0,24 ; 0,16), acide salicylique (0,58 ; 0,34), acide tiglique (0,40 ; 0,33), acide phtalique (0,32 ; 0,21), acide gallique (0,37 ; 0,21) et acide p:hydroxybenzoïque (0,58 ; 0,33), la première valeur de x étant pour un pH final de 7 et la seconde pour un pH final de 7,9.

Suivant l'invention, les compositions proposées
- réduisent les émanations de vapeurs, notammment irritantes, des éventuels autres ingrédients, de départ ou d'arrivée, tel que l'acide acétique,
- sont facilement commercialisables grâce à la stabilité de la formule galénique en poudre, pendant une longue durée, dans des conditions de stockage standardisées,
- permettent, lors de leur usage, de diminuer le temps de contact nécessaire à l'obtention d'un traitement efficace, notamment vis à vis des formes sporulées de bactéries,
- sont efficaces dans les limites prédéfinies, lors de leur usage, grâce à une stabilité suffisante de leurs composants, réduisent au mieux, lors de leur usage, la réactivité de leurs composants avec les métaux ferreux et non ferreux, et
- il est possible de choisir un acide le plus en adéquation avec une réaction des composés peroxydés avec les composées N-acylés devant se faire en milieu alcalin, et capable d'acidifier le milieu réactionnel au pH souhaité qu'une fois la réaction bien engagée.

## Revendications

1. Composition qui est à l'état solide et qui comprend
a) un composé peroxydé,
b) un composé N-acylé apte à réagir en milieu aqueux alcalin avec le composé peroxydé pour donner, par une réaction de perhydrolyse, un peracyl,
**caractérisée en ce qu'**elle comprend
c) un acide tel qu'une solution S telle que définie dans le test A de l'acide en question présente, pour une valeur à l'équilibre du pH donné comprise entre 7 et 7, 9, une différence des pH mesurés à l'instant t = 30 secondes et à l'instant t = 30 minutes qui est supérieure à 0,6.

2. Composition suivant la revendication 1, **caractérisée en ce que** l'acide de c) est un mono ou di-alcénoïque ayant au moins 4 atomes de carbone et solide à la température ambiante, l'acide benzoïque, des dérivés de ceux-ci ou un mélange de ces derniers.

3. Composition suivant la revendication 1 ou 2, **caractérisée en ce que** le composé peroxydé est le perborate de sodium monohydraté.

4. Composition suivant l'une des revendications précédentes, **caractérisée en ce que** le composé N-acylé est la tétraacétyléthylénediamine.

5. Composition suivant l'une des revendications précédentes, **caractérisée en ce que** l'acide de c) est l'acide sorbique.

6. Composition suivant l'une des revendications précédentes, **caractérisée en ce qu'**elle comprend pour une mole de composé peroxydé de 1,5 à 2,5 moles de composé N-acylé.

7. Procédé de préparation d'une composition antimicrobienne **caractérisé en ce qu'**il consiste à verser de 90 à 99 parties en poids d'eau pour une partie en poids de la composition suivant l'une des revendications précédentes dans ladite composition et à attendre pendant au moins 10 minutes et de préférence pendant 15 minutes avant d'utiliser la solution obtenue pour la destruction de l'agent infectieux.

8. Utilisation d'une composition suivant l'une des revendications 1 à 6 pour la destruction d'agent infectieux.
